(19) Europäisches Patentamt / European Patent Office / Office européen des brevets

(11) **EP 4 549 458 A1**

(12) **EUROPEAN PATENT APPLICATION**

(43) Date of publication:
07.05.2025 Bulletin 2025/19

(21) Application number: 24209906.7

(22) Date of filing: **30.10.2024**

(51) International Patent Classification (IPC):
*C07K 14/805* (2006.01)    *A61K 38/42* (2006.01)
*G01N 33/50* (2006.01)    *G01N 33/72* (2006.01)

(52) Cooperative Patent Classification (CPC):
**C07K 14/805; A61K 38/42; G01N 33/96;**
G01N 2496/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.10.2023 JP 2023185918**

(71) Applicant: **ARKRAY, Inc.**
**Kyoto-shi, Kyoto 601-8045 (JP)**

(72) Inventors:
• **AMANO, Chie**
**Kyoto, 602-0008 (JP)**
• **OMOTO, Kazumasa**
**Kyoto, 602-0008 (JP)**

(74) Representative: **Dehns**
**10 Old Bailey**
**London EC4M 7NG (GB)**

(54) **METHOD FOR STABILIZING HEMOGLOBIN A2 AND HEMOGLOBIN F, METHOD OF PRODUCING HEMOGLOBIN SOLUTION, AND HEMOGLOBIN SOLUTION**

(57) A method for stabilizing hemoglobin A2 and hemoglobin F and a method of producing a hemoglobin solution, the methods including preparing a solution containing a buffer, sucrose, and hemoglobin, wherein: the solution has a pH of from 6.0 to 9.6, a concentration of the buffer in the solution is from 5 to 150 mM, a concentration of the hemoglobin in the solution is from 5 to 20 g/L, and the hemoglobin contains at least hemoglobin A2 and hemoglobin F; and a hemoglobin solution, are provided.

EP 4 549 458 A1

**Description**

BACKGROUND

Technical Field

The present disclosure relates to a method for stabilizing hemoglobin A2 and hemoglobin F, a method of producing a hemoglobin solution, and a hemoglobin solution.

Related Art

Precision control substances are used for ensuring test results at clinical examination sites. In general, precision control substances are sold in a freeze-dried state, and are dissolved in purified water or the like for use. Remaining precision control substances are stored by refrigeration, and are used at a later date. Accordingly, precision control substances in liquid form are expected to be stable for a long period.
Japanese Patent Application Laid-Open (JP-A) No. 2016-075504 describes a control sample of hemoglobin (hereinafter, also referred to as "Hb") F, characterized in that sucrose is contained as a stabilizer in a mixed hemolysate containing a hemolysate of human red blood cells and human umbilical cord blood.

SUMMARY OF THE INVENTION

Technical Problem

JP 2016-075504 A describes that there are hemoglobins that are stabilized by sucrose and that are not stabilized by sucrose since different types of hemoglobins have different molecular charges. Accordingly, it seems to be difficult to stabilize different types of hemoglobins simultaneously by simply adding sucrose.
The present disclosure relates to provision of a method for stabilizing HbA2 and HbF, a method of producing an Hb solution, and an Hb solution, in which HbA2 and HbF in an Hb solution can be stabilized.

Solution to Problem

Means for solving the foregoing problem include the following aspects.

(1) A method for stabilizing hemoglobin A2 and hemoglobin F, the method including preparing a solution containing a buffer, sucrose, and hemoglobin, wherein:

the solution has a pH of from 6.0 to 9.6,
a concentration of the buffer in the solution is from 5 to 150 mM,
a concentration of the hemoglobin in the solution is from 5 to 20 g/L, and
the hemoglobin contains at least hemoglobin A2 and hemoglobin F.

(2) The method for stabilizing hemoglobin A2 and hemoglobin F according to (1), wherein a concentration of the sucrose in the solution is from 5 to 20% (w/v).
(3) A method of producing a hemoglobin solution, the method including preparing a solution containing a buffer, sucrose, and hemoglobin, wherein:

the solution has a pH of from 6.0 to 9.6,
a concentration of the buffer in the solution is from 5 to 150 mM,
a concentration of the hemoglobin in the solution is from 5 to 20 g/L, and
the hemoglobin contains at least hemoglobin A2 and hemoglobin F.

(4) The method of producing a hemoglobin solution according to (3), wherein a concentration of the sucrose in the solution is from 5 to 20% (w/v).
(5) A hemoglobin solution, containing a buffer, sucrose, and hemoglobin, wherein:

the solution has a pH of from 6.0 to 9.6,
a concentration of the buffer in the solution is from 5 to 150 mM,
a concentration of the hemoglobin in the solution is from 5 to 20 g/L, and

the hemoglobin contains at least hemoglobin A2 and hemoglobin F.

(6) The hemoglobin solution according to (5), wherein a concentration of the sucrose in the solution is from 5 to 20% (w/v).

(7) The hemoglobin solution according to (5) or (6), wherein the hemoglobin solution is used as a precision control substance.

Advantageous Effects of Invention

The present disclosure provides a method for stabilizing HbA2 and HbF, a method of producing an Hb solution, and an Hb solution, in which HbA2 and HbF in an Hb solution can be stabilized.

BRIEF DESCRIPTION OF THE DRAWINGS

FIG. 1 shows a chromatogram of high-performance liquid chromatography performed in Example 1 using 100 mM Tris-HCl (pH 8.2) at an Hb concentration of 10 g/L.
FIG. 2 shows a chromatogram of high-performance liquid chromatography performed in Example 1 using a 250 mM phosphate buffer solution (pH 6.0) at an Hb concentration of 5 g/L.

DETAILED DESCRIPTION OF THE INVENTION

Hereinafter, embodiments of the present disclosure will be described in detail. However, embodiments of the present disclosure are not limited to the following embodiments. Components (including element steps and the like) in the following embodiments are not essential unless otherwise specified. The same applies to numerical values and their ranges, and the numerical values and their ranges do not limit the embodiments of the present disclosure.

In the present disclosure, an element described in a singular form does not eliminate the presence of plural elements as long as there is no technical contradiction, unless otherwise specified.

In the present disclosure, a numerical range indicated using "(from) ... to" includes the numerical values described before and after "to" as the minimum value and the maximum value, respectively.

In numerical ranges described herein in a stepwise manner, the upper limit value or the lower limit value of one numerical range may be replaced with the upper limit value or the lower limit value of another numerical range described in a stepwise manner. Further, in numerical ranges described herein, the upper limit value or the lower limit value of a numerical range may be replaced with a value described in the Examples section.

In the present disclosure, a combination of two or more preferable aspects is a more preferred aspect.

In the present disclosure, the total amount of Hb in a solution is measured by a cyanmethemoglobin method. Specifically, for example, the total amount of Hb can be measured with HEMOGLOBIN TEST WAKO (FUJIFILM Wako Pure Chemical Corporation).

In the present disclosure, the proportions of HbA2 and HbF in Hb are measured based on the peak area ratios obtained by high-performance liquid chromatography.

In the description of embodiments of the present disclosure, pH refers to the pH at 25°C, unless otherwise specified.

<Method for Stabilizing HbA2 and HbF>

A method for stabilizing HbA2 and HbF according to the present disclosure includes preparing a solution containing a buffer, sucrose, and Hb, in which the pH of the solution is from 6.0 to 9.6, the concentration of the buffer in the solution is from 5 to 150 mM, the concentration of the Hb in the solution is from 5 to 20 g/L, and the Hb contains at least HbA2 and HbF. In the present disclosure, the solution containing a buffer, sucrose, and Hb to be prepared is also referred to as "Hb solution".

The inventors have found that, by preparing an Hb solution under the foregoing specific conditions, both HbA2 and HbF can be stabilized even when the Hb solution is stored for a long period. While it is presumed that different types of hemoglobins have different charges and thereby have different stabilizing conditions, it is believed that both HbA2 and HbF hardly change in their charge states in a solution containing sucrose and having a pH, buffer concentration, and Hb concentration within the foregoing ranges, whereby both can be stably present for a long period. Therefore, for example, a precision control substance containing HbA2 and HbF that are stable for a long period can be produced. Further, for example, the precision control substance can be stored by refrigeration from the time of preparation (dissolution) until it is depleted. Therefore, in routinely conducting accuracy management, cost for the accuracy management can be reduced, and processes for preparation at the time of use or for frozen storage in small portions after the preparation can be omitted, whereby ease of handling is also improved. However, it is noted that embodiments of the present disclosure are not limited by the foregoing mechanisms. In the present disclosure, the expression that HbA2 and HbF are "stable" refers to a state in which the variations in the proportions (%) of HbA2 and HbF with respect to the total Hb before and after storage of the Hb solution are small, in other words, the variations are within predetermined ranges.

JP 2016-075504 A describes that sucrose exhibited a stabilizing effect on HbA1c and HbF, whereas no practical stabilizing effect was exhibited on HbA1A by sucrose. Further, it is described that different types of hemoglobins have different charges, that the difference in the charges has a large influence on the manner of detection in high-performance liquid chromatography, and that, while one of the conditions required for an Hb stabilizer is that it does not affect the charge state of the Hb easily, this is difficult to predict from the correlation between the charge state of the Hb and the saccharide molecule used as the stabilizer. On the other hand, the inventors have focused on two types of hemoglobins, namely HbA2 and HbF, and have investigated for a method for stabilizing both HbA2 and HbF for a long period. The method according to the present disclosure is particularly useful in that both HbA2 and HbF can be stabilized for a long period.

(Hemoglobin)

The Hb solution contains at least HbA2 and HbF, and may or may not contain any other Hb, such as HbA0 or HbA1. The Hb solution typically contains other hemoglobins derived from blood, such as HbA0 and HbA1.

The concentration of the Hb in the Hb solution (that is, the total Hb concentration) is from 5 to 20 g/L. From the viewpoint of stabilizing HbA2 and HbF more favorably, the Hb concentration in the Hb solution is preferably from 10 to 20 g/L, more preferably from 10 to 18 g/L. In particular, in a case in which a buffer described below is a phosphate buffer solution, the Hb concentration is preferably from 10 to 20 g/L, more preferably from 10 to 18 g/L. In a case in which the buffer is a Tris buffer solution, the Hb concentration is preferably from 10 to 20 g/L. In a case in which the buffer is a carbonic acid-bicarbonate buffer solution, the Hb concentration is preferably from 10 to 20 g/L.

The origins of the HbA2 and HbF are not particularly limited. Examples thereof include human and non-human animal blood, and human blood is preferable. Examples of the non-human animal include a non-human mammal (monkey, dog, cat, mouse, rat, rabbit, cow, horse, pig, sheep, etc.) and a bird (chicken, quail, etc.).

The HbA2 may be, for example, HbA2 obtained from human peripheral blood. The HbF may be, for example, HbF obtained from human peripheral blood, or human umbilical cord blood, which is rich in HbF. The origins of the HbA2 and the HbF may be the same sample, or may be separate samples. It is simple to prepare the Hb solution containing HbA2 and HbF from one sample. Further, by a method of mixing HbA2 and HbF obtained from separate samples allows for adjusting the concentrations and proportions of HbA2 and HbF easily.

For example, the HbA2 and HbF may be obtained by hemolyzing human or non-human animal blood to extract Hb. The hemolysis may be performed by, for example, adding EDTA to a precipitate obtained by centrifugation of whole blood obtained by blood drawing, and freezing and thawing the resultant. The HbA2 and HbF may also be obtained by purifying the solution obtained by hemolysis as described above by dialysis or the like.

(Buffer)

The Hb solution contains a buffer. Examples of the buffer include a phosphate buffer solution, a Tris buffer solution (e.g., Tris-HCl, TBS, TBS(-)-T, TAE, TBE, or TE), and a carbonic acid-bicarbonate buffer solution. From the viewpoint of stabilizing HbA2 and HbF more favorably, a Tris buffer solution is preferable, and Tris-HCl is more preferable.

The concentration of the buffer in the Hb solution is from 5 to 150 mM. From the viewpoint of stabilizing HbA2 and HbF more favorably, the concentration is preferably from 5 to 100 mM, more preferably from 5 to 50 mM, still more preferably from 5 to 30 mM. In particular, in a case in which the buffer is a phosphate buffer solution, the concentration of the buffer is preferably from 5 to 100 mM, more preferably from 5 to 50 mM. In a case in which the buffer is a Tris buffer solution, the concentration of the buffer is preferably from 5 to 100 mM. In a case in which the buffer is a carbonic acid-bicarbonate buffer solution, the concentration of the buffer is preferably from 5 to 100 mM, more preferably from 5 to 50 mM, still more preferably from 5 to 30 mM.

(Sucrose)

The Hb solution contains sucrose. The sucrose serves as a stabilizer of Hb. The concentration of the sucrose in the solution is preferably from 5 to 20% (w/v), more preferably from 6 to 18% (w/v), still more preferably from 7 to 15% (w/v).

The amount of the sucrose is preferably from 5 to 20 times, more preferably from 6 to 18 times, still more preferably from 7 to 15 times the amount of the Hb in the Hb solution on a mass basis.

(Hb Solution)

The pH of the Hb solution is from 6.0 to 9.6. From the viewpoint of stabilizing HbA2 and HbF more favorably, the pH of the Hb solution is preferably from 7.0 to 9.2, more preferably from 7.5 to 9.0.

From the viewpoint of stabilizing HbA2 and HbF more favorably, it is preferable that the pH of the Hb solution is from 7.0 to 9.2 (more preferably from 7.0 to 9.0), that the concentration of the buffer is from 5 to 150 mM (preferably from 5 to 100 mM, more preferably from 5 to 50 mM), and that the Hb concentration is from 10 to 20 g/L (preferably from 10 to 18 g/L).

The method for stabilizing HbA2 and HbF in this embodiment includes preparing a solution containing a buffer, sucrose, and Hb. The preparation method is not particularly limited, and the buffer, the sucrose, and the Hb may be mixed by any method. In one aspect, the Hb solution can be prepared by mixing a buffer, a sucrose solution, and a solution containing Hb including HbA2 and HbF. In another aspect, the Hb solution can be prepared by mixing a buffer, a sucrose solution, a solution containing HbA2, and a solution containing HbF. The order of the mixing is not particularly limited.

The prepared Hb solution is preferably stable for 21 days or more, for example, when the solution is stored at 8°C after preparation. For example, the variable value of the proportion of the HbA2 in the Hb solution after 21 days of storage at 8°C after preparation is preferably less than 0.25%, more preferably less than 0.21%. The variable value of the proportion of the HbF in the Hb solution after 21 days of storage at 8°C after preparation is preferably less than 0.60%, more preferably less than 0.40%, still more preferably less than 0.36%. The "variable value" of the proportion of the HbA2 or the proportion of the HbF is determined as follows. The proportions (%) of each of the HbA2 and the HbF in the Hb solution immediately after the preparation and after the storage are determined by high-performance liquid chromatography. The absolute difference between the proportion after the storage and the proportion before the storage is determined, and defined as the variable value of each of the proportions (%) of HbA2 and HbF. Conditions of the high-performance liquid chromatography in the measurement of the variable value are the same as or equivalent to the method described in Example 1.

<Method of Producing Hemoglobin Solution>

A method of producing an Hb solution in an embodiment of the present disclosure includes preparing a solution containing a buffer, sucrose, and Hb, in which the pH of the solution is from 6.0 to 9.6, the concentration of the buffer in the solution is from 5 to 150 mM, the concentration of the Hb in the solution is from 5 to 20 g/L, and the Hb contains at least HbA2 and HbF. The content described in the section "Method for Stabilizing HbA2 and HbF" can apply to the details of the buffer, the sucrose, the Hb, the Hb solution, and the preparation methods thereof in this embodiment.

<Hemoglobin Solution>

An Hb solution in an embodiment of the present disclosure is an Hb solution containing a buffer, sucrose, and Hb, in which the solution has a pH of from 6.0 to 9.6, the concentration of the buffer in the solution is from 5 to 150 mM, the concentration of the Hb in the solution is from 5 to 20 g/L, and the Hb contains at least HbA2 and HbF. The content described in the section "Method for Sabilizing HbA2 and HbF" can apply to the details of the buffer, the sucrose, the Hb, the Hb solution, and the preparation methods thereof in this embodiment.

In one aspect, the Hb solution according to this embodiment is used as a precision control substance. A precision control substance is a sample having a known composition, which is used for accuracy management of analyses, and the precision control substance in this aspect refers to a sample in which the compositions of HbA2 and HbF (that is, the proportions (%) thereof with respect to the total Hb in the precision control substance) are known.

Examples

Next, embodiments of the present disclosure are specifically described with reference to the Examples. However, embodiments of the present disclosure are not limited to these Examples.

1. Preparation of Hb Solution

<Hemolysis>

(1) Human whole blood was centrifuged (10000 g, 10 minutes, 4°C), and the supernatant was removed.
(2) The precipitate was suspended with the same amount of saline as the whole blood.
(3) The suspension from the procedure (2) was centrifuged (10000 g, 10 minutes, 4°C), and the supernatant was removed.

(4) Procedures (2) and (3) were repeated.

(5) The precipitate was suspended with 20 mM EDTA-NaOH (pH 7.0) that is 1.3 times the amount of the whole blood.

(6) The solution from the procedure (5) was frozen at -80°C.

<Purification>

(7) After confirming that the solution from the procedure (6) was completely frozen, the solution was thawed in a water bath at room temperature (about 25°C). The resultant is referred to as "hemolysis specimen".

(8) The hemolysis specimen was placed in a dialysis membrane having a molecular weight cutoff of 3500, and dialyzed with purified water as an external fluid.

(9) The external fluid was exchanged (volume of external fluid: 85 times or more the volume of the internal fluid, 3 hours or more/time, 6 times).

(10) The internal fluid recovered was centrifuged (40000 g, 10 minutes, 4°C), and the supernatant was recovered. The supernatant is referred to as "solution A".

(11) The Hb concentration in the solution A was measured with HEMOGLOBIN TEST WAKO (FUJIFILM Wako Pure Chemical Corporation) (cyanmethemoglobin method).

(12) A 28% (w/v) sucrose solution, a 0.5 M buffer solution, and the solution A that has been quantified in the procedure (11) were mixed such that the final concentrations of the components were adjusted to the designated concentrations.

2. Evaluation

[Example 1]

<Test Conditions>

- Phosphate buffer solution: 5 to 250 mM (pH 6.0, 7.0, 7.5, 8.0, 8.2)
- Tris-HCl: 5 to 250 mM (pH 7.5, 8.0, 8.2, 9.0)
- Carbonic acid-bicarbonate buffer solution: from 5 to 250 mM (pH 9.2, 9.6)
- Final concentration of sucrose: 7% (w/v)
- Hb concentration: from 5 to 20 g/L

<High-Performance Liquid Chromatography>

(1) The proportions of HbA2 and HbF in each specimen were measured (n = 3) using a calibrated high-performance liquid chromatography apparatus.

(2) The specimens were stored at 8°C for 21 days.

(3) The proportions of HbA2 and HbF in each specimen after the storage were measured (n = 3) using the calibrated high-performance liquid chromatography apparatus.

(4) The variable value of the average values of the measurement values (n = 3) of each of the HbA2 proportion (%) and the HbF proportion (%) before and after the storage was calculated.

<High-Performance Liquid Chromatography Apparatus>

(1) High-performance liquid chromatography apparatus

A commercially available cation exchange chromatography column filled with a hydrophilic polymer composed of a methacrylic acid ester copolymer was connected to a commercially available high-performance liquid chromatography apparatus. An optical detector (specifically, absorption spectrometer) for detecting the concentration of the Hb that flows in the flow path was attached at a predetermined position of the flow path downstream of the cation exchange chromatography column.

(2) Eluent

Three types of eluents were prepared as aqueous solutions having the compositions shown in Table 1 below.

[Table 1]

| Raw material | Concentration (% by mass) | | |
|---|---|---|---|
| | Eluent A | Eluent B | Eluent C |
| Sodium dihydrogen phosphate dihydrate | 1.44 | 0.02 | 0.12 |
| Disodium hydrogen phosphate | 0.16 | 0.50 | 0.33 |

The eluent A was adjusted so as to have a pH of 5.08, the eluent B was adjusted so as to have a pH of 8.0, and the eluent C was adjusted so as to have a pH of 6.82. The eluent A had the minimum elution power of Hb and the eluent B had the maximum elution power of Hb, among these eluents.

(3) Chromatogram

The eluent A was allowed to flow in the high-performance liquid chromatography apparatus described in (1) to equilibrate the column. Thereafter, the hemolyzed blood specimen was introduced into the column. Then, the eluent A was allowed to flow for 13 seconds, thereby eluting HbA1a, HbA1b, HbF, and HbA1c. Next, a mixture containing the eluent A and the eluent C in a 1:9 ratio was allowed to flow for 5 seconds, thereby eluting HbA0. Next, the eluent C was allowed to flow for 5 seconds, thereby eluting HbA2. The eluent B was allowed to flow for 2 seconds, thereby eluting the entire Hb remaining in the column, and thereafter the eluent A was allowed to flow for 5 seconds. During the elution, a chromatogram was generated from the absorbance detected by the optical detector at a detection wavelength of 420 nm.

The proportion (%) of HbA2 and the proportion (%) of HbF were determined by the following expressions.

$$\text{HbA2 (\%)} = \text{Total area of HbA2/Total area of total Hb} \times 100$$

$$\text{HbF (\%)} = \text{Total area of HbF/Total area of total Hb} \times 100$$

<Calculation of Variable Value>

The proportion (%) of HbA2 was determined before and after storage of the specimen at 8°C for 21 days. The absolute value between the average value after the storage (n = 3) and the average value before the storage (n = 3) was determined, and was defined as the variable value of the proportion (%) of HbA2. The variable value of the proportion (%) of HbF was also determined by the same method.

<Results>

- The variable values of the proportion (%) of each of HbA2 and HbF are shown in the following Tables. In the Tables, "ND" means that no detection result was obtained owing to the peak being outside the measurement range, although the measurement was performed. "-" means that no measurement was performed. The standard range of the variable value of the proportion (%) of HbA2 was set to less than 0.25%. The standard range of the variable value of the proportion (%) of HbF was set to less than 0.60%.

[Table 2]

| HbA2 phosphate buffer solution | PH 6.0 | | | | | | PH 7.0 | | | | | | PH 7.5 | | | | | | PH 8.0 | | | | | | PH 8.2 | | | | | |
|---|---|---|---|---|---|---|---|---|---|---|---|---|---|---|---|---|---|---|---|---|---|---|---|---|---|---|---|---|---|---|
| Buffer solution concentration (mM) | 5 | 10 | 25 | 50 | 100 | 250 | 5 | 10 | 25 | 50 | 100 | 250 | 5 | 10 | 25 | 50 | 100 | 250 | 5 | 10 | 25 | 50 | 100 | 250 | 5 | 10 | 25 | 50 | 100 | 250 |
| Hb 20 g/L | 0.09 | 0.08 | - | - | 0.22 | - | 0.06 | 0.07 | - | - | 0.13 | - | 0.04 | 0.05 | - | - | 0.11 | - | 0.06 | 0.03 | - | - | 0.07 | - | 0.04 | 0.02 | - | - | 0.08 | - |
| Hb 18 g/L | 0.15 | 0.06 | - | 0.12 | 0.10 | - | 0.08 | 0.10 | - | - | 0.03 | - | 0.05 | 0.06 | - | - | 0.12 | - | 0.03 | 0.05 | - | - | 0.09 | - | 0.04 | 0.02 | - | - | 0.07 | - |
| Hb 10 g/L | 0.07 | 0.10 | - | - | 0.11 | 0.39 | 0.06 | 0.07 | - | - | 0.12 | 0.13 | 0.05 | 0.05 | - | 0.04 | 0.09 | 0.11 | 0.03 | 0.04 | - | 0.06 | 0.06 | 0.04 | 0.03 | 0.05 | - | 0.05 | 0.05 | 0.08 |
| Hb 5 g/L | 0.08 | 0.09 | - | 0.08 | 0.19 | ND | 0.04 | 0.05 | 0.01 | 0.02 | 0.12 | ND | 0.01 | 0.03 | 0.04 | 0.05 | 0.06 | ND | 0.03 | 0.02 | 0.04 | 0.06 | 0.03 | ND | 0.03 | 0.03 | 0.06 | 0.06 | 0.02 | ND |

8

[Table 3]

| HbA2 | Tris buffer solution | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| | pH 7.5 | | | | | | | pH 8.0 | | | | | | | pH 8.2 | | | | | | | pH 9.0 | | | | | | |
| Buffer solution concentration (mM) | 5 | 10 | 30 | 50 | 100 | 150 | 250 | 5 | 10 | 30 | 50 | 100 | 150 | 250 | 5 | 10 | 30 | 50 | 100 | 150 | 250 | 5 | 10 | 30 | 50 | 100 | 150 | 250 |
| Hb 20 g/L | 0.05 | 0.02 | - | - | 0.06 | - | 0.07 | 0.04 | 0.01 | - | - | 0.04 | - | 0.07 | 0.01 | 0.01 | - | - | 0.02 | - | 0.05 | 0.07 | 0.03 | - | - | 0.02 | 0.06 | 0.01 |
| Hb 18 g/L | 0.12 | 0.03 | - | - | 0.02 | - | 0.01 | 0.03 | 0.02 | - | - | 0.01 | - | 0.05 | 0.02 | 0.01 | - | - | 0.01 | - | 0.01 | 0.05 | 0.02 | 0.08 | 0.10 | 0.01 | 0.06 | 0.00 |
| Hb 13 g/L | 0.05 | - | - | - | - | 0.01 | - | 0.08 | - | - | - | - | 0.01 | - | 0.05 | - | - | - | - | 0.06 | - | 0.02 | - | - | 0.07 | 0.02 | - | - |
| Hb 10 g/L | 0.02 | 0.04 | - | - | 0.05 | - | 0.05 | 0.00 | 0.04 | 0.09 | 0.08 | 0.03 | - | 0.04 | 0.02 | 0.01 | 0.09 | 0.07 | 0.02 | - | 0.03 | 0.05 | 0.03 | 0.08 | - | 0.00 | - | 0.07 |
| Hb 5 g/L | 0.03 | 0.01 | 0.07 | 0.07 | 0.00 | 0.07 | 0.01 | 0.02 | 0.02 | 0.10 | 0.10 | 0.01 | - | 0.01 | 0.02 | 0.01 | 0.11 | 0.13 | 0.02 | - | 0.01 | 0.09 | - | 0.13 | 0.12 | - | - | - |

[Table 4]

| HbA2 carbonic acid-bicarbonate buffer solution | PH 9.2 | | | | | | PH 9.6 | | | | | |
|---|---|---|---|---|---|---|---|---|---|---|---|---|
| Buffer solution concentration (mM) | 5 | 10 | 30 | 50 | 100 | 250 | 5 | 10 | 30 | 50 | 100 | 250 |
| Hb 20 g/L | 0.00 | 0.01 | - | 0.09 | 0.01 | 0.02 | 0.01 | 0.01 | - | 0.10 | 0.06 | 0.12 |
| Hb 18 g/L | 0.03 | 0.02 | - | 0.12 | 0.02 | 0.02 | 0.01 | 0.02 | - | 0.12 | 0.03 | 0.11 |
| Hb 10 g/L | 0.01 | 0.01 | 0.05 | 0.12 | 0.01 | 0.02 | 0.00 | 0.01 | 0.00 | 0.16 | 0.08 | 0.25 |
| Hb 5 g/L | 0.01 | 0.02 | 0.07 | 0.09 | 0.04 | 0.16 | 0.01 | 0.01 | 0.07 | 0.16 | 0.16 | ND |

[Table 5]

| HbF phosphate buffer solution | PH 6.0 | | | | | PH 7.0 | | | | | | PH 7.5 | | | | | | PH 8.0 | | | | | | PH 8.2 | | | | | |
|---|---|---|---|---|---|---|---|---|---|---|---|---|---|---|---|---|---|---|---|---|---|---|---|---|---|---|---|---|---|
| Buffer solution concentration (mM) | 5 | 10 | 50 | 100 | 250 | 5 | 10 | 25 | 50 | 100 | 250 | 5 | 10 | 25 | 50 | 100 | 250 | 5 | 10 | 25 | 50 | 100 | 250 | 5 | 10 | 25 | 50 | 100 | 250 |
| Hb 20 g/L | 0.09 | 0.10 | - | 0.08 | - | 0.08 | 0.06 | - | - | 0.12 | - | 0.06 | 0.06 | - | - | 0.13 | - | 0.07 | 0.06 | - | - | 0.11 | - | 0.06 | 0.07 | - | - | 0.08 | - |
| Hb 18 g/L | 0.08 | 0.10 | 0.12 | 0.12 | - | 0.06 | 0.06 | - | - | 0.14 | - | 0.06 | 0.06 | - | - | 0.14 | - | 0.05 | 0.05 | - | - | 0.12 | - | 0.05 | 0.07 | - | - | 0.10 | - |
| Hb 10 g/L | 0.08 | 0.09 | - | 0.12 | 0.06 | 0.10 | 0.07 | - | - | 0.16 | 0.55 | 0.09 | 0.04 | - | 0.15 | - | 0.31 | 0.07 | 0.02 | - | 0.09 | - | 0.29 | 0.06 | 0.03 | - | 0.04 | 0.16 | 0.33 |
| Hb 5 g/L | 0.10 | 0.09 | 0.14 | 0.07 | 0.07 | 0.08 | 0.09 | 0.12 | 0.22 | 0.58 | 0.05 | 0.07 | 0.04 | 0.11 | 0.26 | 0.19 | 8.06 | 0.05 | 0.02 | 0.05 | 0.17 | 0.09 | 1.07 | 0.04 | 0.03 | 0.05 | 0.20 | 0.56 | 0.85 |

[Table 6]

| HbF Tris buffer solution | PH 7.5 | | | | | | | PH 8.0 | | | | | | | PH 8.2 | | | | | | | PH 9.0 | | | | | | |
|---|---|---|---|---|---|---|---|---|---|---|---|---|---|---|---|---|---|---|---|---|---|---|---|---|---|---|---|---|
| Buffer solution concentration (mM) | 5 | 10 | 30 | 50 | 100 | 150 | 250 | 5 | 10 | 30 | 50 | 100 | 150 | 250 | 5 | 10 | 30 | 50 | 100 | 150 | 250 | 5 | 10 | 30 | 50 | 100 | 150 | 250 |
| Hb 20 g/L | 0.09 | 0.08 | - | - | 0.05 | - | 0.04 | 0.06 | 0.04 | - | - | 0.02 | - | 0.03 | 0.05 | 0.02 | - | - | 0.00 | - | 0.04 | 0.03 | 0.01 | - | - | 0.01 | 0.01 | 0.01 |
| Hb 18 g/L | 0.07 | 0.07 | - | - | 0.05 | - | 0.03 | 0.05 | 0.05 | - | - | 0.01 | - | 0.05 | 0.06 | 0.04 | - | - | 0.00 | - | 0.04 | 0.01 | 0.02 | 0.02 | 0.03 | 0.02 | 0.05 | 0.04 |
| Hb 13 g/L | 0.05 | - | - | - | - | 0.04 | - | 0.08 | - | - | - | - | 0.01 | - | 0.05 | - | - | - | - | 0.01 | - | 0.02 | - | - | 0.03 | 0.05 | - | - |
| Hb 10 g/L | 0.05 | 0.03 | - | - | 0.02 | - | 0.05 | 0.02 | 0.04 | 0.02 | 0.02 | 0.01 | - | 0.05 | 0.01 | 0.01 | 0.02 | 0.04 | 0.00 | - | 0.06 | 0.01 | 0.01 | 0.04 | - | 0.02 | - | 0.16 |
| Hb 5 g/L | 0.04 | 0.03 | 0.02 | 0.07 | 0.05 | - | 0.21 | 0.00 | 0.01 | 0.02 | 0.02 | 0.04 | - | 0.04 | 0.02 | 0.01 | 0.02 | 0.01 | 0.05 | - | 0.05 | 0.05 | - | 0.06 | 0.03 | - | - | - |

[Table 7]

| HbF carbonic acid-bicarbonate buffer solution | PH 9.2 | | | | | | PH 9.6 | | | | | |
|---|---|---|---|---|---|---|---|---|---|---|---|---|
| Buffer solution concentration (mM) | 5 | 10 | 30 | 50 | 100 | 250 | 5 | 10 | 30 | 50 | 100 | 250 |
| Hb 20 gIL | 0.03 | 0.01 | - | 0.04 | 0.02 | 0.04 | 0.03 | 0.02 | - | 0.07 | 0.03 | 0.03 |
| Hb 18 g/L | 0.02 | 0.02 | - | 0.05 | 0.02 | 0.01 | 0.01 | 0.01 | - | 0.06 | 0.03 | 0.04 |
| Hb 10 g/L | 0.02 | 0.04 | 0.02 | 0.05 | - | - | 0.01 | 0.03 | 0.07 | 0.08 | - | 0.61 |
| Hb 5 g/L | 0.04 | 0.02 | 0.04 | 0.09 | 0.02 | 0.09 | 0.02 | 0.04 | 0.09 | 029 | 0.18 | 0.28 |

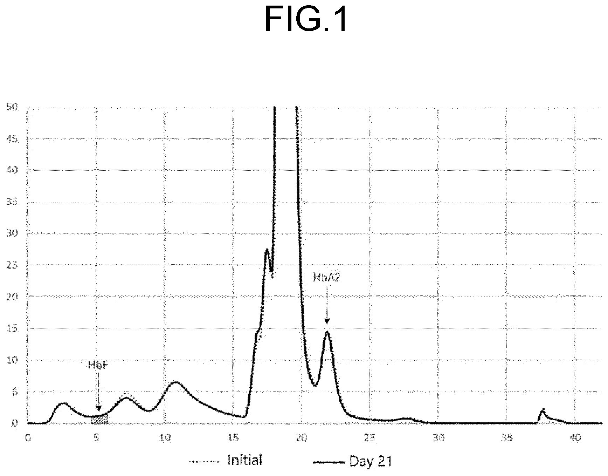

An example of the chromatograms is shown in FIG. 1. FIG. 1 is a chromatogram under conditions of 100 mM Tris-HCl (pH 8.2) and an Hb concentration of 10 g/L. The dotted line indicates a chromatogram on day 0 (the day of preparation), and the solid line indicates a chromatogram on day 21. The peaks of HbA2 and the amounts of HbF on day 0 and day 21 match, suggesting that the specimen is stable.

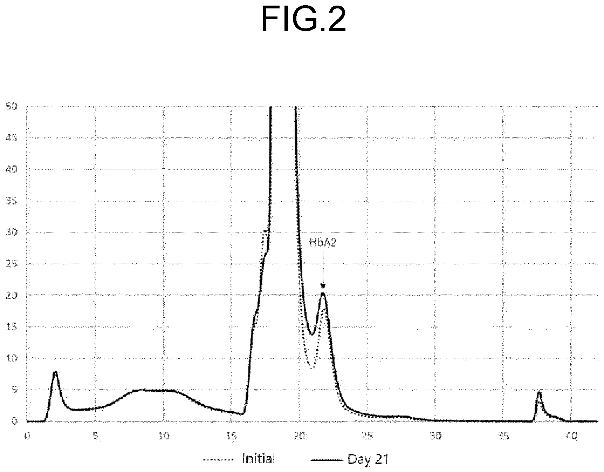

Another example of the chromatograms is illustrated in FIG. 2. FIG. 2 is a chromatogram under conditions of a 250 mM phosphate buffer solution (pH 6.0) and an Hb concentration of 5 g/L. The dotted line indicates a chromatogram on day 0 (the day of preparation), and the solid line indicates a chromatogram on day 21. The peaks of HbA2 on day 0 and day 21 do not match, suggesting that the specimen is not stable.

The Example suggests that HbA2 and HbF in the Hb solutions having a pH of from 6.0 to 9.6, a buffer concentration of from 5 to 150 mM, and an Hb concentration of from 5 to 20 g/L are stable at 8°C for 21 days.

[Example 2]

<Test Conditions>

- Tris buffer solution: 25 mM (pH 8.8)
- Final concentration of sucrose: 7% (w/v)
- Hb concentration: from 13 to 18 g/L

<High-Performance Liquid Chromatography>

(1) The proportions of HbA2 and HbF in each specimen were measured (n = 3) using a calibrated high-performance liquid chromatography apparatus.
(2) The specimens were stored at 8°C for 21 days.
(3) The proportions of HbA2 and HbF in each specimen after the storage were measured (n = 3) using the calibrated high-performance liquid chromatography apparatus.
(4) The variable value of the average values of the measurement values (n = 3) of each of the HbA2 proportion (%) and the HbF proportion (%) before and after the storage was calculated.

<High-Performance Liquid Chromatography Apparatus>

An apparatus ADAMS HA-8180T (manufactured by ARKRAY Inc.) was used as the high-performance liquid chromatography apparatus. A COLUMN UNIT 80T (manufactured by ARKRAY Inc.), which is a column filled with 0.45 ml of a hydrophilic polymer composed of a methacrylic acid ester copolymer as a filler, was used for the apparatus. As reagents, 80A as an eluent A, 80B as an eluent B, and 80CT as an eluent C (manufactured by ARKRAY Inc.), were used for the elution of Hb from the column, and 80H was used as a hemolysis/cleaning solution. The composition and pH of each of 80A, 80B, 80CT, and 80H are as follows. The reagents were allowed to flow in the column at a flow rate of 1.7 ml/min.

13

Eluent A (80A): phosphate buffer solution (pH 5.35 ± 0.05)
Eluent B (80B): phosphate buffer solution (pH 8.05 ± 0.20)
Eluent C (80CT): phosphate buffer solution (pH 7.0 to 7.4)
Hemolysis/cleaning solution (80H): phosphate buffer solution containing a surfactant (pH 7.5 ±0.1)

<Calculation of Variable Value>

The variable values of the proportions (%) of HbA2 and HbF were determined in the same manner as in Example 1.

<Results>

The variable values of the proportion (%) of HbA2 and the proportion (%) of HbF of the specimens are shown in the Table below. The standard range of the variable value of the proportion (%) of HbA2 was set to less than 0.25%. The standard range of the variable value of the proportion (%) of HbF was set to less than 0.60%. The variable values of the proportions of HbA2 and HbF were confirmed to be within the standard ranges.

[Table 8]

| Variable value | Day 21 |
|---|---|
| HbA2 [%] | 0.10 |
| HbF [%] | 0.04 |

## Claims

1. A method for stabilizing hemoglobin A2 and hemoglobin F, the method comprising preparing a solution containing a buffer, sucrose, and hemoglobin, wherein:

   the solution has a pH of from 6.0 to 9.6,
   a concentration of the buffer in the solution is from 5 to 150 mM,
   a concentration of the hemoglobin in the solution is from 5 to 20 g/L, and
   the hemoglobin contains at least hemoglobin A2 and hemoglobin F.

2. The method for stabilizing hemoglobin A2 and hemoglobin F according to claim 1, wherein a concentration of the sucrose in the solution is from 5 to 20% (w/v).

3. A method of producing a hemoglobin solution, the method comprising preparing a solution containing a buffer, sucrose, and hemoglobin, wherein:

   the solution has a pH of from 6.0 to 9.6,
   a concentration of the buffer in the solution is from 5 to 150 mM,
   a concentration of the hemoglobin in the solution is from 5 to 20 g/L, and
   the hemoglobin contains at least hemoglobin A2 and hemoglobin F.

4. The method of producing a hemoglobin solution according to claim 3, wherein a concentration of the sucrose in the solution is from 5 to 20% (w/v).

5. A hemoglobin solution, comprising a buffer, sucrose, and hemoglobin, wherein:

   the solution has a pH of from 6.0 to 9.6,
   a concentration of the buffer in the solution is from 5 to 150 mM,
   a concentration of the hemoglobin in the solution is from 5 to 20 g/L, and
   the hemoglobin contains at least hemoglobin A2 and hemoglobin F.

6. The hemoglobin solution according to claim 5, wherein a concentration of the sucrose in the solution is from 5 to 20% (w/v).

7. The hemoglobin solution according to claim 5 or 6, wherein the hemoglobin solution is used as a precision control substance.

# FIG.1

# FIG.2

Europäisches
Patentamt

European
Patent Office

Office européen
des brevets

# EUROPEAN SEARCH REPORT

Application Number

EP 24 20 9906

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 191 037 A1 (INT REAGENTS CORP [JP]) 27 March 2002 (2002-03-27) * par.8-12, 21, 29, 30 * | 1-7 | INV. C07K14/805 A61K38/42 G01N33/50 |
| Y | JP 2016 075504 A (BML INC) 12 May 2016 (2016-05-12) * par.19-21, 27, 34, 40, 50 * | 1-7 | G01N33/72 |
| Y | CN 107 266 563 A (SICHUAN ORIENTER BIOLOGICAL TECH CO LTD) 20 October 2017 (2017-10-20) * par.6, 20-23, 30 * | 1-7 | |

TECHNICAL FIELDS
SEARCHED      (IPC)

C07K
A61K
G01N

The present search report has been drawn up for all claims

| Place of search | Date of completion of the search | Examiner |
|---|---|---|
| Munich | 3 March 2025 | Bonello, Steve |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 20 9906

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2025

| Patent document cited in search report | | Publication date | Patent family member(s) | | Publication date |
|---|---|---|---|---|---|
| EP 1191037 | A1 | 27-03-2002 | AT | E305013 T1 | 15-10-2005 |
| | | | CA | 2377822 A1 | 11-01-2001 |
| | | | DE | 60022751 T2 | 13-07-2006 |
| | | | EP | 1191037 A1 | 27-03-2002 |
| | | | JP | 5410647 B2 | 05-02-2014 |
| | | | KR | 20020026463 A | 10-04-2002 |
| | | | WO | 0102438 A1 | 11-01-2001 |
| JP 2016075504 | A | 12-05-2016 | JP | 6467182 B2 | 06-02-2019 |
| | | | JP | 2016075504 A | 12-05-2016 |
| CN 107266563 | A | 20-10-2017 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016075504 A